(19) Europäisches Patentamt
European Patent Office
Office européen des brevets

(11) Veröffentlichungsnummer: **0 247 222**
**A1**

# (12) EUROPÄISCHE PATENTANMELDUNG

(21) Anmeldenummer: **86107218.9**

(51) Int. Cl.[4]: **C07D 209/88**

(22) Anmeldetag: **28.05.86**

(43) Veröffentlichungstag der Anmeldung:
**02.12.87 Patentblatt 87/49**

(84) Benannte Vertragsstaaten:
**AT BE CH DE FR GB IT LI NL**

(71) Anmelder: **F. HOFFMANN-LA ROCHE & CO. Aktiengesellschaft**

**CH-4002 Basel(CH)**

(72) Erfinder: **Coffen, David Llewellyn**
**270 Ridgewood Avenue**
**Glen Ridge, N.J. 07028(US)**
Erfinder: **Mandeville, W. Harry**
**7 Pillings Pond Road**
**Lynnfield, Massachusetts 01940(US)**

(74) Vertreter: **Mahé, Jean et al**
**Postfach 3255 Grenzacherstrasse 124**
**CH-4002 Basel(CH)**

## (54) Carbazolderivate.

(57) Die Erfindung betrifft neue Carbazole der Formel

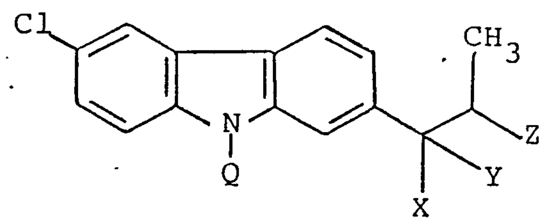

II

worin Q Wasserstoff oder -COR′, R′ Wasserstoff, $C_{1-9}$-Alkyl, Phenyl oder Phenyl substituiert durch Nitro, Halogen, $C_{1-9}$-Alkyl oder Carboxy, X und Y zusammen Sauerstoff und Z Wasserstoff oder Halogen oder jedes von X und Y $C_{1-5}$-Alkoxy und Z Hydroxy oder $OSO_2R''$ und R″ Methyl, Aethyl oder Phenyl p-substituiert durch Chlor, Nitro oder Methyl sind,
sowie Verfahren zu deren Herstellung und zu deren Ueberführung in Carprofen.

EP 0 247 222 A1

## Carbazolderivate

Die Erfindung betrifft ein Verfahren zur Herstellung der 6-Chlor-α-methylcarbazol-2-essigsäure (Carprofen), einer für ihre antiinflammatorische Wirksamkeit bekannten Verbindung, sowie neue in diesem Verfahren verwendbare Carbazolderivate.

Nach dem Verfahren gemäss der Erfindung wird Carbazol in eine Verbindung der Formel

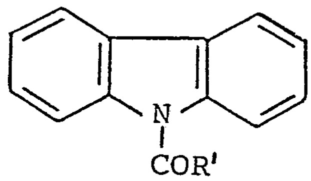

IV

worin R′ Wasserstoff, $C_{1-9}$ -Alkyl, Phenyl oder Phenyl substituiert durch Nitro, Halogen, $C_{1-9}$-Alkyl oder Carboxy,
umgewandelt. $C_{1-9}$ -Alkyl bezeichnet eine geradkettige oder verzweigte Gruppe, wie Methyl, Aethyl, Propyl oder Isopropyl. Das Carbazol kann man mit dem gemischten Anhydrid von Ameisensäure und Essigsäure oder mit Acetanhydrid, Propionsäureanhydrid oder Benzoesäureanhydrid zu 9-Formyl-, 9-Acetyl-, 9-Propionyl-bzw. 9-Benzoylcarbazol umsetzen. Die Reaktion kann man in einem organischen Lösungsmittel wie Methylenchlorid, Schwefelkohlenstoff, Nitrobenzol oder vorzugsweise Tetrachloräthylen, z.B. bei Rückflusstemperatur des verwendeten Lösungsmittels durchführen. Das Produkt der Formel IV kann durch Entfernen des Lösungsmittels und Kristallisation isoliert werden. Eine Verbindung der Formel IV kann in eine Verbindung der Formel

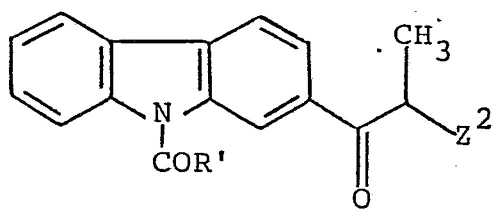

IIIA

worin R′ die obige Bedeutung hat und $Z^2$ Halogen ist, umgewandelt werden, durch Reaktion mit einem Friedel-Crafts-Reagenz, wie wasserfreiem Aluminiumchlorid, und α-Halopropionylchlorid in einem aprotischen Lösungsmittel, wie Nitrobenzol, Schwefelkohlenstoff, Methylenchlorid oder polychloriertes Aethan oder Aethylen, vorzugsweise Tetrachloräthylen, zweckmässig bei Rückflusstemperatur des verwendeten Lösungsmittels.

Eine Verbindung der Formel IIIA kann in eine Verbindung der Formel

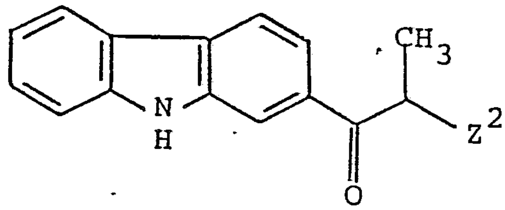

IIIB

worin $Z^2$ die obige Bedeutung hat,
umgewandelt werden durch Reaktion mit einer anorganischen Säure, wie Schwefelsäure oder Salzsäure.

Eine Verbinung der Formel IV kann auch in eine Verbindung der Formel

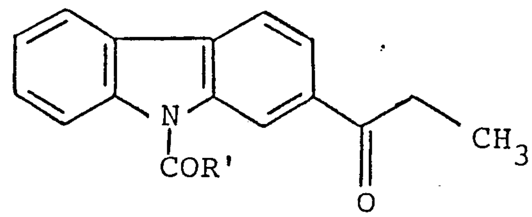

IIIC

worin R' die obige Bedeutung hat, umgewandelt werden, durch Friedel-Crafts-Reaktion wie weiter oben beschrieben, jedoch bei einer Temperatur im Bereich von etwa 15 bis 50°C, vorzugsweise etwa 20°C, und mit Propionylchlorid.

Eine Verbindung der Formel IIIC kann mit einer konzentrierten anorganischen Säure, wie Schwefelsäure, unter Rückfluss in Wasser und einem Alkohol, z.B. Aethanol, und dann Behandlung mit einer Base, z.B. Natriumhydroxid, zu 2-Pro pionylcarbazol hydrolysiert werden.

In einem besonderen Aspekt betrifft die Erfindung die Chlorierung von 2-Propionylcarbazol oder einer Verbindung der Formel IIIA, IIIB oder IIIC, d.h. der Formel

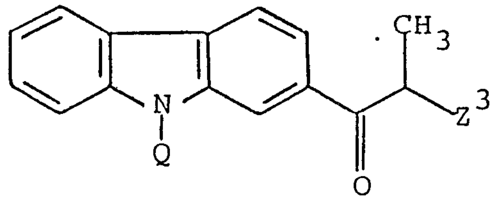

III

worin Q Wasserstoff oder -COR' ist, R' die obige Bedeutung hat und $Z^3$ Wasserstoff oder Halogen ist, zu einer Verbindung der Formel

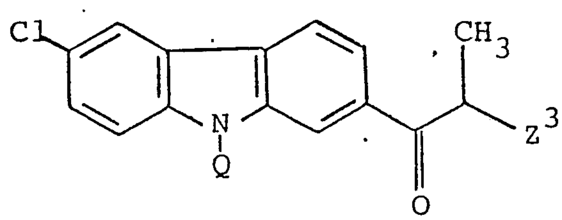

IIE

worin Q und $Z^3$ die obige Bedeutung haben.

Dies kann mit einem Chlorierungsmittel erfolgen aus der Reihe von t-Butylhypochlorit, Chlor, unterchlorige Säure, Sulfurylchlorid, Trichlorisocyanursäure und 1-Chlorbenzotriazol, in einem polaren organischen Lösungsmittel aus der Gruppe von 1,2-Dichloräthan, Aethylacetat, Trimethylphosphat, Triäthylphosphat, Dimethylacetamid, Dimethylformamid (DMF), Methylenchlorid und einem Gemisch von DMF und Methylenchlorid.

Vorzugsweise geht man von N-acylierten Verbindungen der Formel III aus, verwendet Sulfurylchlorid oder Trichlorisocyanursäure als Chlorierungsmittel und DMF oder Methylenchlorid als Lösungsmittel. Anorganische Säuren, wie Salzsäure, Bromwasserstoffsäure, Schwefelsäure oder Phosphorsäure, vorzugsweise gasförmige Chlorwasserstoffsäure, kann man als Katalysatoren verwenden. Die Reaktion kann man bei einer Temperatur von etwa 40°C oder unterhalb dieser Temperatur durchführen. Die Verbindungen der Formel IIE kann man in an sich bekannter Weise, z.B. durch Kristallisation isolieren. Eine N-unsubstituierte Verbindung kann man zu einer Verbindung der Formel IIE acylieren, worin Q -COR' ist, in der gleichen Weise wie oben beschrieben für die Herstellung der Verbindung der Formel IV.

In einem weiteren Aspekt betrifft die Erfindung ein Verfahren zur Herstellung einer Verbindung der Formel

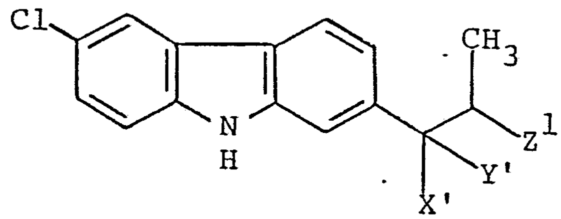

IIC

worin jedes von X′ und Y′ $C_{1-5}$-Alkoxy, $Z^1$ Hydroxy oder $OSO_2R''$ und R″ Methyl, Aethyl oder Phenyl p-substituiert durch Chlor, Nitro oder Methyl,
das dadurch gekennzeichnet ist, dass man

a) eine Verbindung der Formel

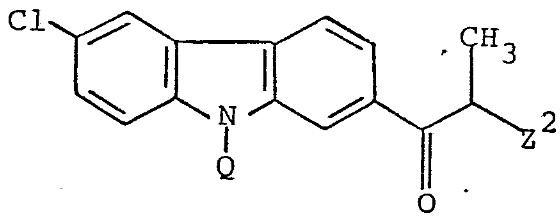

IID

worin Q und $Z^2$ die obige Bedeutung haben,
mit einem Alkali-oder Erdalkalimetall gelöst in einem $C_{1-5}$-Alkohol umsetzt, und

b) erwünschtenfalls eine erhaltene Verbindung der Formel IIC, worin $Z^1$ Hydroxy ist, mit einem Sulfonylchlorid $R''SO_2Cl$, worin R″ die obige Bedeutung hat, umsetzt.

Vorzugsweise wird die Reaktion a) mit Natrium in Methanol bei Raumtemperatur und die Reaktion b) in einem aprotischen Lösungsmittel, wie Toluol, Aethylacetat oder Methylenchlorid in Gegenwart einer Base, z.B. Pyridin, durchgeführt. Pyridin kann auch als Lösungsmittel verwendet werden.

In einem weiteren Aspekt betrifft die Erfindung ein Verfahren zur Herstellung einer Verbindung der Formel

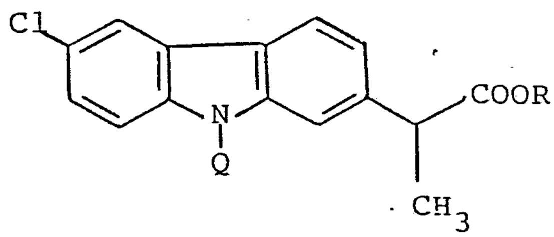

I

worin Q die obige Bedeutung hat und R $C_{1-5}$-Alkyl ist,
das dadurch gekennzeichnet ist, dass man

a) eine Verbindung der Formel

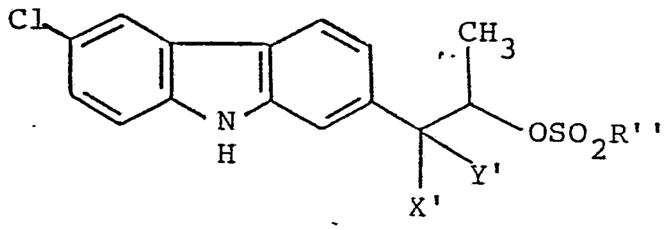

IIA

worin X′, Y′ und R″ die obige Bedeutung haben,
mit einem Puffer in Wasser und einem polaren organischen Lösungsmittel umsetzt, oder

b) eine Verbindung der Formel

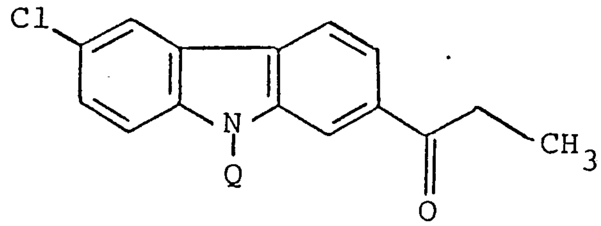

IIB

worin Q obige Bedeutung hat, mit einem Thallium(III)Salz und einem Trialkylorthoester oder einem $C_{1-5}$ -Alkohol oder einem Gemisch eines Trialkylorthoesters und eines $C_{1-5}$-Alkohols umsetzt.

Als Puffer in der solvolytischen Umwandlung a) einer Verbindung der Formel IIA zu einer solchen der Formel I kann man ein Erdalkalimetallcarbonat oder Hydroxid, z.B. Barium oder Calciumcarbonat oder -Hydroxid, oder Magnesiumhydroxid, oder ein Alkalimetallcarbonat oder Bicarbonat, z.B. Natrium-, Kalium- oder Lithiumcarbonat oder Bicarbonat, oder Aluminiumhydroxid verwenden. Als polares organisches Lösungsmittel kann man ein $C_{1-5}$-Alkohol oder Alkandiol, z.B. Aethanol, Aethylenglykol oder vorzugsweise Methanol, Wasser, Tetrahydrofuran, DMF, Dimethylsulfoxid oder Dioxan verwenden. Die Reaktion kann man bei einer Temperatur bis zur Rückflusstemperatur des verwendeten Lösungsmittels, vorzugsweise bei etwa 60-65°C durchführen.

Der erhaltene Ester der Formel I, worin Q Wasserstoff ist, kann in situ zu Carprofen hydrolysiert werden mit einem wässrigen Alkalimetallhydroxid, z.B. Natrium-oder Kaliumhydroxid, in Gegenwart eines Lösungsmittels, wie einem Alkohol, z.B. Methanol oder Aethanol, oder mit einer wässrigen Säure, z.B. Salzsäure oder Schwefelsäure, bei einer Temperatur bis zur Rückflusstemperatur des Reaktionsgemisches. Das Carprofen kann man durch Entfernen des Lösungsmittels, Verdünnung mit Wasser, Filtrierung zur Entfernung von in Basen unlöslichem Material und Fällung durch Zusatz einer starken Säure, z.B. Salzsäure, isolieren. Das Produkt kann man über sein Salz mit einem Alkylamin, z.B. Triäthylamin, reinigen.

Als Thalliumsalz in der Reaktion b) kann man das Trinitrat, als Trialkylester, das Triäthyl-oder Trimethylorthoacetat oder Orthoformat, vorzugsweise das Trimethylorthoformat verwenden. Zusätzlich zum Alkohol, z.B. Methanol, Aethanol, Propanol oder Butanol kann man ein anderes Lösungsmittel verwenden, z.B. Acetonitril oder Methylenchlorid oder ein Gemisch davon, und die Reaktion kann man bei einer Temperatur zwischen etwa 0°C oder Raumtemperatur bis zur Rückflusstemperatur des verwendeten Lösungsmittels, z.B. etwa 40-42°C durchführen. Das Produkt kann man durch Abfiltrieren des als Nebenprodukt gebildeten Thalliumsalzes isolieren.

Die erhaltene Verbindung der Formel I, worin Q COR′ ist und R′ die obige Bedeutung hat, kann man zu Carprofen hydrolysieren, entweder mittels einer wässrigen Base, z.B. Natrium-oder Kaliumhydroxid, in einem Alkohol, z.B. Methanol oder Aethanol, unter Rückfluss, gefolgt durch Zusatz einer starken Säure, z.B. Salzsäure, oder direkt in saurem Medium, z.B. in Salzsäure oder in einem Gemisch davon mit Essigsäure.

Die Verbindungen der Formeln IIB, insbesondere das 6-Chlor-2-propionylcarbazol oder 9-Acetyl-6-chlor-2-propionylcarbazol, IIC, insbesondere das 2-Hydroxy-1,1-dimethoxy-1-(6-chlor-9H-carbazol-2-yl)propan oder 2-Methansulfonyloxy-1,1-dimethoxy-1-(6-chlor-9H-carbazol -2-yl)propan, und IID, insbesondere das 2-Chlor-1-(6-chlor-9H-carbazol-2-yl)-1-propanon oder 2-Chlor-1-(6-chlor-9-acetylcarbazol-2-yl)-1-propanon, d.h. die Verbindungen der Formel

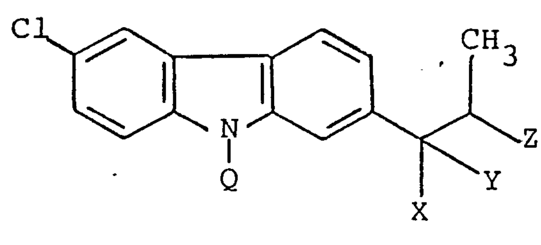

II

worin X und Y zusammen Sauerstoff und Z Wasserstoff oder Halogen oder jedes von X und Y $C_{1-5}$-Alkoxy und Z Hydroxy oder $OSO_2R''$ sind und R″ und Q die obige Bedeutung haben, sind neu und als solche Gegenstand der Erfindung.

Beispiel 1

a) Ein Gemisch von 83,6 g Carbazol, 1400 ml Tetrachloräthylen und 56,25 g Essigsäureanhydrid in denen 1,0 g kon zentrierte Schwefelsäure gelöst war, wurde vier Stunden unter Rückfluss und dann über Nacht bei Raumtempratur gerührt. Ein Volumen von 400 ml wurde abdestilliert, gesammelt und mit 200 ml 3N Natriumhydroxid zum Entfernen der Essigsäure geschüttelt und zur Verwendung bei der Verarbeitung aufbewahrt. Das Reaktionsgemisch wurde auf 50°C abgekühlt. Unter Rühren wurden 200 g pulverförmiges wasserfreies Aluminiumchlorid gefolgt durch 90 g 2-Chlorpropionylchlorid zugesetzt. Das Reaktionsgemisch wurde auf 90°C erhitzt. Dann wurde es auf 50°C abgekühlt. Dann wurde 1 kg zerkleinertes Eis zugesetzt. Nach 2 1/2-stündigem Rühren wurde die wässrige Schicht mit der Hälfte des aufbewahrten Tetrachloräthylendestillats extrahiert. Die vereinigten organischen Schichten wurden über wasserfreiem Natriumsulfat getrocknet und filtriert. Mit dem Rest des Destillats wurde der Filterkuchen gewaschen.

Das Lösungsmittel wurde von dem Filtrat entfernt, wobei man 1250 ml Tetrachloräthylen zurückgewann. Durch Kristallisation des Rückstandes erhielt man 2-Chlor-1-(9-acetyl-carbazol-2-yl)-1-propanon. Der feste Kuchen wurde zerkleinert und weiteres Lösungsmittel wurde unter Vakuum entfernt. Man erhielt 200 g (Theorie 150 g) Produkt. 200 mg davon wurden aus Methylenchlorid/Aether/Hexan umkristallisiert, Smp. 103-106°C. Der Rest wurde in der nächsten Stufe direkt verwendet.

b) Die 200 g Produkt des Beispiels 1a) wurden in 350 ml DMF aufgeschlämmt. Dann wurden 50 g Trichlorisocyansäure zugesetzt und das Gemisch wurde zwei Stunden gerührt. Während ein paar Sekunden wurde trockenes gasförmiges Chlorwasserstoff in den Luftraum über der gebildeten Lösung eingeblasen Durch Abkühlen im Wasserbad wurde die Temperatur auf 40°C gehalten. Das Reaktionsgemisch wurde über Nacht belassen. Das kristallisierte Produkt wurde filtriert und der Filterkuchen wurde mit dem Filtrat gewaschen. Nach Lufttrocknen erhielt man 124 g Material, die für die Umkristallisation beibehalten wurden. Von dem Filtrat wurden 250 ml DMF zurückgewonnen. Der Rückstand wurde mit 500 ml warmem Wasser zerrieben. Nach einer Stunde wurde das Material filtriert, mit Wasser gewaschen und luftgetrocknet. Dieses Material wurde in 450 ml heisser Essigsäure aufgenommen und zur Entfernung von unlöslichem Material filtriert. Der Filterkuchen wurde mit 50 ml heisser Essigsäure gewaschen. Das Filtrat wurde über Nacht belassen und das zusätzliche auskristallisierte Produkt wurde durch Filtrierung gesammelt, mit 50 ml Essigsäure gewaschen und luftgetrocknet. Das Produkt wurde in 350 ml kochender Essigsäure zugesetzt. Die Lösung wurde zur Entfernung von unlöslichem Material filtriert und der Filterkuchen wurde mit 50 ml kochender Essigsäure gewaschen. Die vereinigten Filtrate wurde zur Auflösung des Materials erhitzt. Die Lösung wurde abgekühlt. Das kristallisierte Produkt wurde durch Filtrierung gesammelt, mit 100 ml Essigsäure gewaschen und luftgetrocknet. Man erhielt 111,4 g (66,7%) 2-Chlor-1-(6-chlor-9-acetyl-carbazol-1-yl)-1-propanon, Smp. 155-158°C.

Beispiel 2

111,15 g 2-Chlor-1-(6-chlor-9-acetyl-carbazol-2-yl)--1-propanon wurden unter Rühren einer Lösung von 33,4 g Natrium in 900 ml Methanol bei 35°C zugesetzt. Dann wurden 100 ml Methanol dem Reaktionsgemsich zugesetzt und das Rühren wurde zwei Stunden fortgesetzt. 300 ml Wasser wurden dann unter Rühren zugesetzt. Nach zwei Stunden wurde das Produkt filtriert, der Filterkuchen wurde mit Wasser gewaschen und luftgetrocknet. Man erhielt 103,75 g (97,4%) 2-Hydroxy-1,1-dimethoxy-1-(6-chlor-9H-carbazol-2-yl)-propan, Smp. 115-120°C (Zersetzung) nach Umkristallisation aus Aether/Hexan.

Beispiel 3

Zu 103,75 g ds Produktes von Beispiel 2 in 1000 ml Methylenchlorid wurden unter Rühren 100 ml Pyridin gefolgt durch 50 g Methansulfonylchlorid zugesetzt. Die Lösung wurde zwei Stunden bei Raumtemperatur gerührt. Zusätzliche 25 g Methansulfonylchlorid und nach drei Stunden zusätzliche 25 g Methansulfonylchlorid und 50 ml Pyridin wurden zugesetzt. Das Gemisch wurde über Nacht gerührt. Die Lösung wurde mit 4 x 500 ml kaltem Wasser gewaschen. Jede Wasserschicht wurde mit der gleichen 100 ml Portion Methylenchlorid rückextrahiert. Die vereinigten organischen Schichten wurden über wasserfreiem Natriumsulfat getrocknet und filtriert. 790 ml Methylenchlorid wurden unter Vakuum zurückgewonnen. Zurückbleibendes Pyridin wurde unter Vakuum entfernt. Man erhielt 147,25 g (Theorie 129,1 g) 1,1-Dimethoxy-2-methansulfonyloxy-1-(6-chlor -9H-carboazol-2-yl)propan, die in der nächsten Stufe verwendet wurden.

Beispiel 4

Dem Produkt von Beispiel 3 gelöst in 1500 ml Methanol wurden 300 ml heisses Wasser und 150 g Calciumcarbonat zugesetzt. Das Gemisch wurde unter Rückfluss gerührt. Dem entstandenen 6-Chlor-α-methyl-carbazol-2-essigsäuremethylester wurde über eine Zeitspanne von 40 Minuten eine Lösung von 56,1 g Kaliumhydroxid in 200 ml Wasser unter Rühren unter Rückfluss zugetropft. Nach 1 1/2 Stunden wurde das Gemisch abgekühlt und 1200 ml Methanol wurden entfernt. Der Rückstand wurde mit 1000 ml heissem Wasser verdünnt und filtriert. Der Filterkuchen wurde mit 2 x 500 ml heissem Wasser gewaschen. Die vereinigten Filtrate wurden abgekühlt und mit 500 ml Aether extrahiert. Die Aetherschicht wurde mit 100 ml Wasser extrahiert. Die vereinigten wässrigen Schichten wurden mit 100 ml konzentrierter Salzsäure angesäuert. Das Produkt wurde durch Filtrierung gesammelt, mit Wasser gewaschen und über Nacht luftgetrocknet. Man erhielt 60,75 g 6-Chlor-α-methyl-carbazol-2-essigsäure.

Beispiel 5

a) Ein Gemisch von 501,6 g Carbazol, 336,9 g Essigsäureanhydrid, 10,0 g konzentrierter Schwefelsäure und 300 ml Methylenchlorid wurden 40 Stunden unter Rückfluss erhitzt. Das Reaktionsgemisch wurde auf Raumtemperatur abgekühlt, dann in eine Lösung von 303 g Natriumbicarbonat in 3 l Wasser geschüttet. Die organische Phase wurde getrennt, mit 3 l Wasser gewaschen und über wasserfreiem Natriumsulfat getrocknet. Durch Destillation wurden 2500 ml Methylenchlorid entfernt. 5000 ml Aethanol wurden zugesetzt und die Destillation wurde fortgesetzt, bis die Dampftemperatur 75°C erreichte. Der Kolben wurde dann auf 0 bis -5°C abgekühlt und eine Stunde gerührt. Durch Filtrierung erhielt man 589,3 g (94%) 9-Acetylcarbazol. Auf analoger Weise wurden 513,1 g (77%) 9-Propionylcarbazol hergestellt.

b) Einer Lösung von 20,9 g 9-Acetylcarbazol und 15,25 g α-Chlorpropionylchlorid in 200 ml Methylenchlorid wurden 33,5 g wasserfreies Aluminiumchlorid unter Rühren zugesetzt. Das Gemisch wurde dann auf Rückfluss während sechs Stunden erhitzt und bei Raumtemperatur über Nacht gerührt. Dem Reaktionsgemisch wurden 200 ml Aethanol gefolgt durch 400 ml 6N Salzsäure zugesetzt. Die organische Schicht und ein Methylenchloridextrakt der wässrigen Schicht wurden kombiniert, getrocknet und eingedampft. Das resultierende Oel wurde in 100 ml Aethanol enthaltend 10 ml konzentrierte Schwefelsäure gelöst. Das Gemisch wurde 1 1/2 Stunden im Dampfbad erhitzt und dann abgekühlt. Das Produkt wurde durch Filtrierung gesammelt, mit Aethanol gewaschen und luftgetrocknet. Man erhielt 25,0 g (97%) 2-Chlor-1-(9H-carbazol--2-yl)-1-propanon in Form von gelben Kristallen.

c) Einer gekühlten Lösung von 5,0 g 2-Chlor-1-(9H-carbazol-2-yl)-1-propanon in 200 ml Methylenchlorid und 3 ml DMF wurden unter Rühren 1,87 ml Sulfurylchlorid zugesetzt. Nach 15 Minuten wurde die Lösung mit 2 x 50 ml Wasser gewaschen, getrocknet und mit Aktivkohle behandelt. Die filtrierte Lösung wurde auf etwa 50 ml konzentriert und abgekühlt. Man erhielt 3,7 g gelbe Kristalle von 2-Chlor-1-(6-chlor-9H-carbazol-2-yl)-1-propanon.

d) Einer Lösung von 0,5 g Natrium in 10 ml Methanol wurden 0,5 g 2-Chlor-1-(6-chlor-9H-carbazol-2-yl)-1-propanon zugesetzt. Das Gemisch wurde zwei Stunden bei Raumtemperatur gerührt. Wasser wurde zugesetzt und das Gemisch wurde mit Methylenchlorid extrahiert. Die vereinigten Extrakte wurden getrocknet und zu einem Gummi eingedampft, der aus Aether kristallisierte. Der Aether wurde eingedampft. Das Produkt, 2-Hydroxy-1,1-dimethoxy-1-(6-chloro-9H-carbazol-2-yl)propan, wurde direkt in der nächsten Stufe, d.h. in Beispiel 3 verwendet.

Beispiel 6

a) Ein Gemisch von 223,3 g 9-Acetylcarbazol, 333,4 g Aluminiumchlorid, 101,8 g Propionylchlorid und 3250 ml Methylenchlorid wurde 16 Stunden bei Raumtemperatur gerührt. Das Reaktionsgemisch wurde mit 1625 ml einer Lösung von 50% Aethanol in Wasser zugesetzt. Die organische Schicht wurde abgetrennt und über wasserfreiem Natriumsulfat getrocknet. Das Gemisch wurde destilliert, dann auf 0 bis -5°C abgekühlt und eine Stunde gerührt. Durch Filtrierung erhielt man 229 g (86%) 9-Acetyl-2-propionylcarbazol. In analoger Weise wurde 2,9-Dipropionylcarbazol aus 9-Propionylcarbazol hergestellt.

b) Ein Gemisch von 263,3 g (9-Acetyl-2-propionylcarbazol, 100 g konzentrierte Schwefelsäure, 2300 ml Aethanol und 230 ml Wasser wurde 18 Stunden unter Rückfluss erhitzt. Das Gemisch wurde heiss filtriert und die warme Lösung wurde unter Zusatz einer Lösung von 80 g Natriumhydroxid und 2170 ml

Wasser gerührt. Das Gemisch wurde sechs Stunden bei Raumtempartur gerührt. Der Feststoff wurde filtriert und in 6000 ml Wasser gewaschen. Durch Filtrierung erhielt man 212 g 2-Propionylcarbazol das auch ausgehend von 2,9-Dipropionylcarbazol hergestellt werden kann.

c) 70,1 g Sulfurylchlorid wurden tropfenweise einer gerührten Suspension von 111,7 g 2-Propionylcarbazol in 2200 ml Methylenchlorid zugetropft. Das Gemisch wurde eine Stunde unter Rückfluss erhitzt. Filtrierung des Feststoffs und Umkristallisation aus 1000 ml 9% DMF - 91% Xylol ergab 54,1 g 6-Chlor-2-propionylcarbazol.

d) Ein Gemisch von 71,2 g 6-Chlor-2-propionylcarbazol und 282 g Essigsäureanhydrid wurde 1 1/2 Stunden unter Rückfluss erhitzt. Nach einer halben Stunde wurden 99.4 ml Wasser der Lösung zugetropft. Dann wurde die Lösung auf Raumtemperatur abgekühlt und über Nacht gerührt. Filtrierung ergab 63,2 g (76%) 9-Acetyl-6-chlor-2-propionylcarbazol.

e) Einer Lösung von 22,2 g Thalliumtrinitrattrihydrat in 19,1 g Trimethylorthoformat wurden unter Rühren 111 ml Acetonitril, 222 ml Methylenchlorid und 15,0 g 9-Acetyl-6-chlor-2-propionylcarbazol zugesetzt und die resultierende Lösung wurde über Nacht gerührt. Das Reaktionsgemisch wurde dann filtriert und die Lösungsmittel wurden abgedampft. Kristallisierung des Rückstandes aus Diäthyläther und Hexan ergab 11,6 g (70%) 9-Acetyl-6-chlor-α-methylcarbazol-2-essigsäuremethylester.

f) Ein Gemisch von 8,24 g 9-Acetyl-6-chlor-α-methylcarbazol-2-essigsäuremethylester, 8,0 g Natriumhydroxid, 100 ml Wasser und 50 ml Aethanol wurde 20 Stunden unter Rückfluss erhitzt. 25 ml konzentrierte Salzsäure und 200 ml Wasser wurden der heissen Lösung zugesetzt. Die resultierende Aufschlämmung wurde auf Raumtemperatur abgekühlt und filtriert. Der resultierende Feststoff wurde mit Wasser gewaschen und getrocknet. Man erhielt 6,8 g (100%) 6-Chlor-α-methylcarbazol-2-essigsäure. Dieses Produkt wurde in einer Lösung von 2,0 g Natriumhydroxid in 100 ml heissem Wasser gelöst. Die resultierende Lösung wurde filtriert und dann mit 4 ml konzentrierter Salzsäure angesäuert. Die Kristalle wurden filtriert, mit Wasser gewaschen und luftgetrocknet. Man erhielt 6,0 g (88%) 6-Chlor-α-methylcarbazol-2-essigsäure. Das Produkt wurde in 180 ml unter Rückfluss erhitztem Toluol gelöst. Die resultierende Lösung wurde mit 1,8 g Aktivkohle entfärbt und filtriert. Kühlen auf -10 bis -5°C und Filtrierung ergaben 4,5 g (66%) 6-Chlor-α-methylcarbazol-2-essigsäure.

## Ansprüche

1. Verfahren zur Herstellung einer Verbindung der Formel

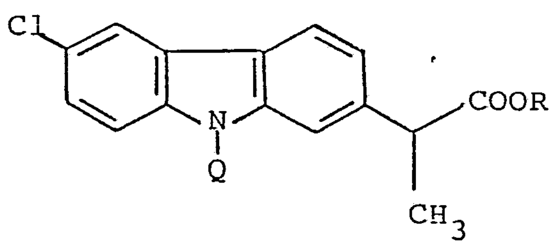

I

worin Q Wasserstoff oder -COR′, R′ Wasserstoff, $C_{1-9}$-Alkyl, Phenyl oder Phenyl substituiert durch Nitro, Halogen, $C_{1-9}$ -Alkyl oder Carboxy und R $C_{1-5}$-Alkyl ist,
dadurch gekennzeichnet, dass man

a) eine Verbindung der Formel

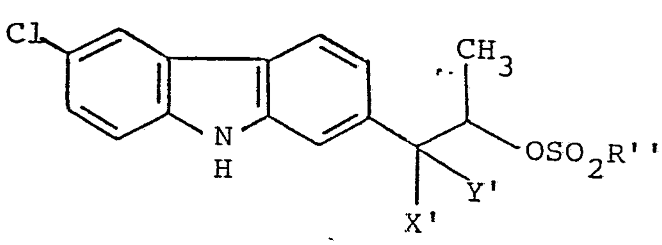

IIA

worin jedes von X′ und Y′ $C_{1-5}$-Alkoxy und R″ Methyl, Aethyl, Phenyl p-substituiert durch Chlor, Nitro oder Methyl ist,
mit einem Puffer in Wasser und einem polaren organischen Lösungsmittel umsetzt, oder

b) eine Verbindung der Formel

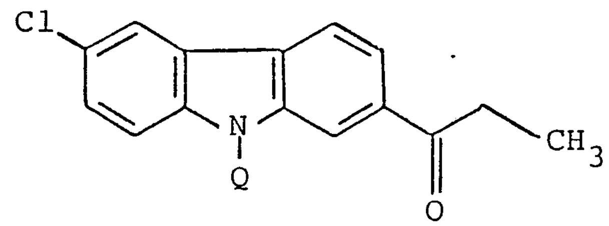

IIB

worin Q obige Bedeutung hat, mit einem Thallium(III)Salz und einem Trialkylorthoester oder einem $C_{1-5}$-Alkohol oder einem Gemisch eines Trialkylorthoesters und eines $C_{1-5}$-Alkohols umsetzt.

2. Verfahren zur Herstellung einer Verbindung der Formel

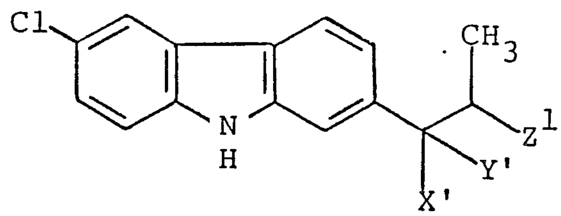

IIC

worin jedes von X' und Y' $C_{1-5}$-Alkoxy, $Z^1$ Hydroxy oder $OSO_2R''$ und R'' Methyl, Aethyl oder Phenyl p-substituiert durch Chlor, Nitro oder Methyl,
dadurch gekennzeichnet, dass man

a) eine Verbindung der Formel

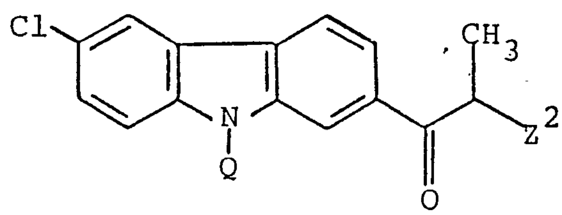

IID

worin Q Wasserstoff oder -COR', R' Wasserstoff, $C_{1-9}$-Alkyl, Phenyl oder Phenyl substituiert durch Nitro, Halogen, $C_{1-9}$-Alkyl oder Carboxy, und $Z^2$ Halogen ist,
mit einem Alkalimetall oder Erdalkalimetall gelöst in einem $C_{1-5}$-Alkohol umsetzt, und

b) erwünschtenfalls eine erhaltene Verbindung der Formel IIC, worin $Z^1$ Hydroxy ist, mit einem Sulfonylchlorid $R''SO_2Cl$ worin R'' die obige Bedeutung hat, umsetzt.

3. Verfahren zur Herstellung einer Verbindung der Formel

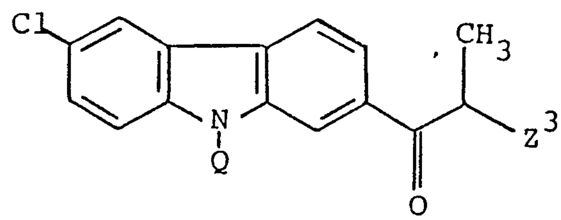

IIE

worin Q Wasserstoff oder -COR', R' Wasserstoff, $C_{1-9}$-Alkyl, Phenyl oder Phenyl substituiert durch Nitro, Halogen, $C_{1-9}$-Alkyl oder Carboxy und $Z^3$ Wasserstoff oder Halogen ist,
dadurch gekennzeichnet, dass man eine Verbindung der Formel

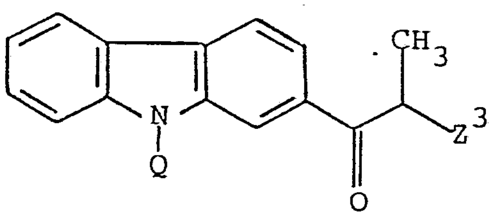

III

worin Q und $Z^3$ die obige Bedeutung haben, mit einem Chlorierungsmittel aus der Gruppe von t-Butylhypochlorid, Chlor, unterchlorige Säure, Sulfurylchlorid, Trichlorisocyanursäure und 1-Chlorbenzotriazol in einem polaren organischen Lösungsmittel aus der Gruppe von 1,2-Dichloräthan, Aethylacetat, Trimethylphosphat, Triäthylphosphat, Dimethylacetamid, Dimethylformamid, Methylenchlorid und einem Gemisch von Dimethylformamid und Methylenchlorid umsetzt.

4. Carbazolderivate der Formel

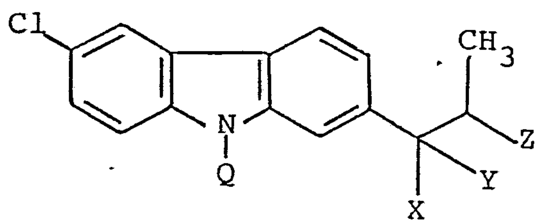

II

worin Q Wasserstoff oder -COR′, R′ Wasserstoff, $C_{1-9}$-Alkyl, Phenyl oder Phenyl substituiert durch Nitro, Halogen, $C_{1-9}$-Alkyl oder Carboxy, X und Y zusammen Sauerstoff und Z Wasserstoff oder Halogen oder jedes von X und Y $C_{1-5}$-Alkoxy und Z Hydroxy oder $OSO_2R''$ und R″ Methyl, Aethyl oder Phenyl p-substituiert durch Chlor, Nitro oder Methyl sind.

5. Eine Verbindung nach Anspruch 4 und der Formel

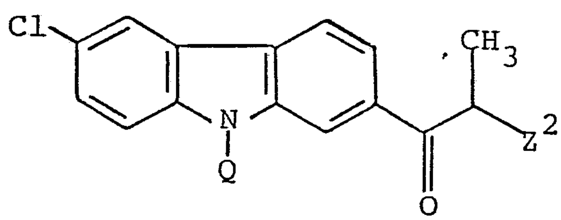

IID

worin Q die gleiche Bedeutung wie in Anspruch 4 hat und $Z^2$ Halogen, insbesondere Chlor ist.

6. Eine Verbindung gemäss Anspruch 4 oder 5, 2-Chlor-1-(6-chlor-9H-carbazol-2-yl)-1-propanon oder 2-Chlor-1-(6-chlor-9-acetylcarbazol-2-yl)-1-propanon.

7. Eine Verbindung nach Anspruch 4 und der Formel

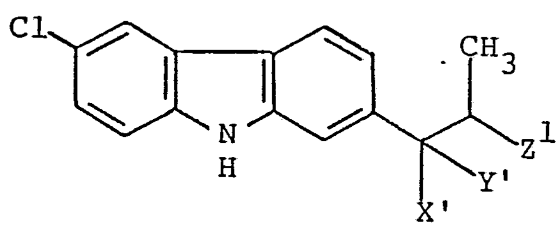

IIC

worin jedes von X′ und Y′ $C_{1-5}$-Alkoxy, $Z^1$ Hydroxy oder $OSO_2R''$ und R″ die gleiche Bedeutung wie in Anspruch 4 hat, insbesondere Methyl oder Aethyl.

8. Eine Verbindung nach Anspruch 4 oder 7, 2-Hydroxy-1,1-dimethoxy-1-(6-chlor-9H-carbazol-2-yl)-propan oder 2-Methansulfonyloxy-1,1-dimethoxy-1-(6-chlor -9H-carbazol-2-yl)propan.

9. Eine Verbindung nach Anspruch 4 und der Formel

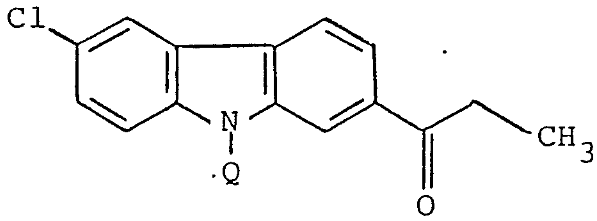

IIB

worin Q die gleiche Bedeutung wie in Anspruch 4 hat, insbesondere -COR', worin R' $C_{1-9}$-Alkyl ist.

10. Eine Verbindung nach Anspruch 4 oder 9, 6-Chlor-2-propionylcarbazol oder 9-Acetyl-6-chlor-2-propionylcarbazol.

Europäisches Patentamt

# EUROPÄISCHER RECHERCHENBERICHT

Nummer der Anmeldung

EP 86 10 7218

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP-A-0 064 394 (SYNTEX PHARMACEUTICALS INTERNATIONAL LTD.)<br>* Seite 3, Zeile 30 - Seite 7, Zeile 29; Seite 9, Zeilen 6-34; Seite 10, Zeilen 23-34, in Zusammenhang mit Seite 2, Zeilen 24-26; Seite 12, Zeile 25 - Seite 19, Zeile 26 *<br>--- | 1,2,4-8 | C 07 D 209/88 |
| Y | EP-A-0 101 124 (ZAMBON S.P.A.)<br>* Seite 3, Zeilen 28-30, Seite 4, Zeile 3 - Seite 5, Zeile 2, besonders Seite 4, Zeile 18 *<br>--- | 1,2,4-8 | |
| Y | EP-A-0 048 136 (SAGAMI CHEMICAL RESEARCH CENTER)<br>* Seite 3, Zeile 30 - Seite 10, Zeile 37; Seite 15, Zeile 2 - Seite 19, Zeile 3 *<br>--- | 1,2,4-8 | RECHERCHIERTE SACHGEBIETE (Int. Cl.4)<br>C 07 D 209/00<br>C 07 C 51/00<br>C 07 C 67/00 |
| Y | JOURNAL OF THE AMERICAN CHEMICAL SOCIETY, Band 98, Nr. 10, 12. Mai 1976, Seiten 3037-3038; E.C. TAYLOR et al.: "Novel Oxidative Rearrangements with Thallium(III) Nitrate(TTN) in Trimethyl Orthoformate(TMOF)"<br>* Seite 3038 - Spalte 1, Zeilen 15-21 *<br>--- -/- | 1,3-6, 9,10 | |

Der vorliegende Recherchenbericht wurde für alle Patentansprüche erstellt.

| Recherchenort | Abschlußdatum der Recherche | Prüfer |
|---|---|---|
| BERLIN | 16-01-1987 | VAN AMSTERDAM L.J.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

Europäisches Patentamt

# EUROPÄISCHER RECHERCHENBERICHT

Nummer der Anmeldung

EP 86 10 7218

Seite 2

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| Y | CHEMICAL ABSTRACTS, Band 60, Nr. 5, 2. März 1964, Spalte 5435h - 5436 d, Columbus, Ohio, US; Spalte 5436, Zeilen 28, 29, 32-34; & PHARMAZIE, Band 18, Nr. 7, 1963, Seiten 456-460<br>--- | 1,3-6, 9,10 | |
| Y | EP-A-0 151 423 (F. HOFFMANN-LA ROCHE & CO.)<br>* Seite 4, Zeile 13 - Seite 5, Zeile 1 *<br>--- | 1,3-6, 9,10 | |
| A | PATENT ABSTRACTS OF JAPAN, Band 10, Nr. 85 (C-336)[2142], 4. April 1986; & JP - A - 60 218 332 (KYOWA HAKKO KOGYO K.K.) 01.11.1985<br>----- | 7 | RECHERCHIERTE SACHGEBIETE (Int. Cl.4) |

Der vorliegende Recherchenbericht wurde für alle Patentansprüche erstellt.

| Recherchenort | Abschlußdatum der Recherche | Prüfer |
|---|---|---|
| BERLIN | 16-01-1987 | VAN AMSTERDAM L.J.P. |

KATEGORIE DER GENANNTEN DOKUMENTE
- X : von besonderer Bedeutung allein betrachtet
- Y : von besonderer Bedeutung in Verbindung mit einer anderen Veröffentlichung derselben Kategorie
- A : technologischer Hintergrund
- O : nichtschriftliche Offenbarung
- P : Zwischenliteratur
- T : der Erfindung zugrunde liegende Theorien oder Grundsätze
- E : älteres Patentdokument, das jedoch erst am oder nach dem Anmeldedatum veröffentlicht worden ist
- D : in der Anmeldung angeführtes Dokument
- L : aus andern Gründen angeführtes Dokument
- & : Mitglied der gleichen Patentfamilie, übereinstimmendes Dokument